# EUROPÄISCHE PATENTSCHRIFT

(11) **EP 3 150 170 B1**
(45) Veröffentlichungstag und Bekanntmachung des Hinweises auf die Patenterteilung: **06.12.2017**
(21) Anmeldenummer: 15188049.9
(22) Anmeldetag: 02.10.2015
(51) Int. Cl.: A61F 2/16

(54) **MULTIFOKALE LINSE UND VERFAHREN ZU DEREN HERSTELLUNG**
MULTIFOCAL LENS AND METHOD OF MANUFACTURING THE SAME
LENTILLE MULTIFOCALE ET SON PROCÉDÉ DE FABRICATION

(43) Veröffentlichungstag der Anmeldung: 05.04.2017
(73) Patentinhaber: Rayner Intraocular Lenses Limited, Sackville Road Hove East Sussex BN3 7AN (GB)
(72) Erfinder: LUX, Kirsten, 2340 Mödling (AT); PLANK, Nicole, 7212 Forchtenstein (AT); BREZNA, Wolfgang, 2700 Wiener Neustadt (AT); DRAGOSTINOFF, Nikolaus, 1230 Wien (AT)
(74) Vertreter: SR Huebner - Munich Patentanwaltspartnerschaft mbB

(56) Entgegenhaltungen:
- US-A1- 2011 267 693

## Beschreibung

Die vorliegende Erfindung betrifft eine multifokale Intraokular- oder Kontaktlinse, mit einem refraktiven Brennpunkt und einer ersten und einer zweiten diffraktiven Struktur, die sich zumindest teilweise überlagern. In einem weiteren Aspekt betrifft die Erfindung ein Verfahren zur Herstellung einer solchen multifokalen Linse.

Multifokale Intraokular- oder Kontaktlinsen, d.h. Linsen mit mehreren Brennpunkten, die z.B. für Nah- und Fernsicht (bifokal) oder Nah-, Intermediär- und Fernsicht (trifokal) verwendet werden können, sind seit mehreren Jahrzehnten bekannt und verwenden verschiedenste diffraktive Strukturen auf einer refraktiven Basislinse, um zusätzlich zum refraktiven Brennpunkt einen oder mehrere diffraktive Brennpunkte zu schaffen.

Gemäß den Schriften DE 20 2009 018 881 U1 und EP 2 503 962 B1 werden dazu zwei diffraktive Strukturen mit jeweils Kinoformprofil überlagert. Die eine der beiden diffraktiven Strukturen hat hier einen Brennpunkt erster Ordnung, der mit dem Brennpunkt zweiter Ordnung der anderen Struktur zusammenfällt. Die Anmelderin hat erkannt, dass die Berechnung solcher Strukturen äußerst kompliziert ist. Zudem führt eine Einarbeitung solcher Strukturen in die Linse zu einer Vielzahl schwierig zu fertigender Profilspitzen, was wiederum zu einer suboptimalen Verteilung bzw. Lichtausbeute der Lichtintensitäten in den erzeugten Brennpunkten führt. Aus dem Dokument US-A-2011/0267693 ist eine multifokale Intraokular- oder Kontaktlinse gemäß dem Oberbegriff des Anspruchs 1 bekannt. Die Erfindung setzt sich zum Ziel, eine verbesserte Linse zu schaffen, die die Nachteile des Standes der Technik überwindet.

Gemäß einem ersten Aspekt der Erfindung wird das Ziel mit einer Linse der einleitend genannten Art erreicht, bei welcher die beiden diffraktiven Strukturen unterschiedlich sind, und wobei ein Brennpunkt erster Ordnung der ersten diffraktiven Struktur mit einem Brennpunkt erster Ordnung der zweiten diffraktiven Struktur zusammenfällt.

Durch die Überlagerung zweier diffraktiver Strukturen, deren Brennpunkte erster Ordnung zusammenfallen, kann die Berechnung der überlagerten diffraktiven Struktur erheblich vereinfacht werden, da die zu überlagernden Strukturen jeweils ein Profil mit gleichen Periodenlängen haben. Adjustierungen der Strukturen zur Ermittlung einer optimalen Intensitätsverteilung während der Berechnung können somit besonders einfach durchgeführt werden.

Bevorzugt haben die beiden diffraktiven Strukturen unterschiedliche Intensitätsverteilungen, wodurch eine individuelle Mischung von Intensitätsverhältnissen möglich ist.

Eine besonders vorteilhafte Ausführungsform der Erfindung zeichnet sich dadurch aus, dass die erste diffraktive Struktur eine um den refraktiven Brennpunkt asymmetrische Intensitätsverteilung und die zweite diffraktive Struktur eine um den refraktiven Brennpunkt im Wesentlichen symmetrische Intensitätsverteilung hat.

Durch die Überlagerung dieser beiden diffraktiven Strukturen wird eine Linse geschaffen, deren für Nah-, Intermediär- und Fernsicht nutzbare Brennpunkte einen höheren Intensitätsanteil aufweisen als im Stand der Technik bekannt. Zur genaueren Betrachtung des Problems werden im Folgenden diffraktive Brennpunkte "positiver" Ordnung als diejenigen definiert, die zwischen der Linse und ihrem refraktiven Brennpunkt liegen, und diffraktive Brennpunkte "negativer" Ordnung als diejenigen, die auf der der Linse abgewandten Seite des refraktiven Brennpunkts liegen.

Wenn beispielsweise der refraktive Brennpunkt für die Fernsicht eingesetzt wird, entspricht der Brennpunkt erster positiver Ordnung der diffraktiven Strukturen einem Abstand für die Intermediärsicht und der Brennpunkt zweiter positiver Ordnung der diffraktiven Strukturen einem Abstand für die Nahsicht. Die jeweiligen negativen Brennpunkte der diffraktiven Strukturen werden in diesem Fall erst hinter der Netzhaut des Benutzers der Linse abgebildet, weshalb diese für den Benutzer nicht nutzbar sind und zu einer Verschlechterung der Bildqualität beitragen.

Durch die erfindungsgemäße Überlagerung einer diffraktiven Struktur mit symmetrischer Intensitätsverteilung mit einer diffraktiven Struktur mit asymmetrischer Intensitätsverteilung werden hingegen Intensitätsanteile der (ursprünglich) negativen Ordnungen auf die genutzten positiven Ordnungen bzw. auf die nullte (refraktive) Ordnung abgebildet, wodurch sich im Vergleich zum Stand der Technik ein farbintensiveres und kontrastreicheres Bild ergibt, da die nutzbaren Brennpunkte höhere Intensitätsanteile aufweisen.

Dieselben Vorteile ergeben sich, wenn beispielsweise in einer alternativen Ausführungsform der refraktive Brennpunkt für die Nahsicht eingesetzt wird, und der Brennpunkt erster negativer Ordnung der Überlagerungsstruktur einem Abstand für die Intermediärsicht und der Brennpunkt zweiter negativer Ordnung der Überlagerungsstruktur einem Abstand für die Fernsicht entspricht. In dieser Ausführungsform sind die positiven Ordnungen der diffraktiven Strukturen schlecht nutzbar, da sie vor dem Nahsicht-Brennpunkt liegen, und die Ordnungen dritter negativer Ordnung gar nicht nutzbar, da sie erst hinter der Netzhaut fokussiert werden. Gemäß der Erfindung werden hier die Intensitätsanteile der positiven Ordnungen auf die nullte (refraktive), negative erste und negative zweite Ordnung abgebildet, wodurch sich im Vergleich zum Stand der Technik wieder eine höhere Lichtausbeute in den nutzbaren Brennpunkten und damit ein farbintensiveres und kontrastreicheres Bild ergibt.

Bevorzugt haben die Profile der beiden diffraktiven Strukturen eine gleiche Periodenlänge, wobei das Profil der ersten diffraktiven Struktur innerhalb der Periode monoton steigend ist und das Profil der zweiten diffraktiven Struktur in der ersten Hälfte der Periode ein Minimum und in der zweiten Hälfte der Periode ein Maximum hat. Dadurch wird erreicht, dass das resultierende Profil der Linse eine geringe Anzahl an Profilspitzen aufweist. Derartige Spitzen sind schwierig zu fertigen und führen bei einer ungenauen Fertigung zu Fehlern im Bildbereich, wodurch sich dem Betrachter ein unscharfes bzw. trübes Bild ergibt. Beispielsweise hat die erste diffraktive Struktur, d.h. die Struktur mit der asymmetrischen Intensitätsverteilung, ein Profil mit Sägezahn-, Echelette- oder Kinoform, und die zweite diffraktive Struktur, d.h. die Struktur mit der im Wesentlichen symmetrischen Intensitätsverteilung, ein Profil mit Rechteck-, Trapez- oder Sinusform. Dadurch können Profilverläufe eingesetzt werden, deren Verhalten gut bekannt ist und die zudem eine leichte Fertigung erlauben.

Gemäß einem bevorzugten Merkmal der Erfindung kann zumindest eines der Profile abgerundete oder abgefaste Flanken haben. Dadurch können Fertigungsprozesse mit geringeren Toleranzen gewählt werden, was sowohl Herstellungskosten als auch Herstellungszeit minimiert.

In einer weiteren vorteilhaften Ausführungsform der Erfindung überlagern sich die beiden diffraktiven Strukturen nur in einem zentralen Bereich der Linse. Beispielsweise kann die Linse außerhalb des zentralen Bereichs überhaupt keine diffraktive Struktur aufweisen. Wird die Pupille größer, z.B. bei geringem Lichteinfall, so wird der Einfluss des nicht-zentralen Bereichs bzw. des refraktiven Anteils für die Fernsicht größer, so dass bei solchen Pupillengrößen die Intensitätsverteilung lediglich die Fernsicht umfasst. Alternativ kann die Linse außerhalb des zentralen Bereichs auch nur eine der beiden diffraktiven Strukturen aufweisen, wodurch z.B. die Nah- und Fernsicht oder die Intermediär- und Fernsicht bei weiten Pupillen am größten gestaltet werden kann.

Bevorzugt sind die beiden diffraktiven Strukturen in radialer Richtung der Linse apodisiert, bevorzugt unterschiedlich stark. Dadurch wird erreicht, dass sich bei einer kleinen Pupille eine andere Intensitätsverteilung ergibt als bei einer großen Pupille. Zudem ergibt sich ein stetiger Übergang der Änderung der Intensitätsverteilung bei aufweitenden bzw. verkleinernden Pupillen. Werden die Apodisierungen unterschiedlich stark gewählt, können die Intensitätsverteilungen nach Belieben an die gewünschte Pupillengröße angepasst werden.

Die Linse der Erfindung kann grundsätzlich in beliebigen optischen Geräten eingesetzt werden, besonders eignet sie sich jedoch als multifokale Kontakt- oder Intraokularlinse.

Gemäß einem zweiten Aspekt schafft die Erfindung auch ein Verfahren zur Herstellung der hier vorgestellten Linse. In einer ersten Ausführungsform umfasst das Verfahren die folgenden Schritte:
a) Bereitstellen eines Linsenrohlings mit einem refraktiven Brennpunkt;
b) Berechnen einer Überlagerungsstruktur aus einer ersten diffraktiven Struktur und einer zweiten diffraktiven Struktur, wobei die beiden diffraktiven Strukturen unterschiedlich sind und wobei ein Brennpunkt erster Ordnung der ersten diffraktiven Struktur mit einem Brennpunkt erster Ordnung der zweiten diffraktiven Struktur zusammenfällt; und
c) Einarbeiten der Überlagerungsstruktur in den Linsenrohling zum Herstellen der multifokalen Linse.

Der Schritt des Einarbeitens der Überlagerungsstruktur in den Linsenrohling erfolgt bevorzugt durch Drehen, was eine besonders präzise Fertigung der multifokalen Linse erlaubt.

In einer zweiten Ausführungsform umfasst das Verfahren die folgenden Schritte:
a) Bereitstellen eines Formrohlings;
b) Berechnen einer Überlagerungsstruktur aus einer ersten diffraktiven Struktur und einer zweiten diffraktiven Struktur, wobei die beiden diffraktiven Strukturen unterschiedlich sind und wobei ein Brennpunkt erster Ordnung der ersten diffraktiven Struktur mit einem Brennpunkt erster Ordnung der zweiten diffraktiven Struktur zusammenfällt;
c) Einarbeiten der Überlagerungsstruktur als Negativ in den Formrohling; und
d) Herstellen der multifokalen Linse durch Inkontaktbringen eines Linsenmaterials mit dem Formrohling, wobei dem Linsenmaterial auch ein refraktiver Brennpunkt verliehen wird.

Bei dieser Ausführungsform wird zuerst ein Formrohling mit der Überlagerungsstruktur, d.h. eine "Negativform", geschaffen, z.B. durch Drehen oder Fräsen. Danach kann die Linse mittels der Negativform gegossen, gepresst, geprüft oder sonstwie geformt werden.

Bevorzugt erfolgt das Inkontaktbringen durch Gießen des Linsenmaterials auf den Formrohling und Aushärten des Linsenmaterials. Das Linsenmaterial kann hierbei von selbst erstarren oder z.B. mittels Lichts oder Wärmebehandlung ausgehärtet werden.

Beide Ausführungsformen des erfindungsgemäßen Verfahrens zeichnen sich durch eine einfache Berechnung der Überlagerungsstruktur aus, indem zwei Strukturen z.B. durch einfache Addition überlagert werden, was geringe Rechenleistung benötigt.

Insbesondere bevorzugt hat bei beiden Ausführungsformen die erste diffraktive Struktur eine um den refraktiven Brennpunkt asymmetrische Intensitätsverteilung und die zweite diffraktive Struktur eine um den refraktiven Brennpunkt im Wesentlichen symmetrische Intensitätsverteilung.

Bezüglich weiterer Merkmale und Vorteile des erfindungsgemäßen Verfahrens zur Herstellung der multifokalen Linse wird auf die vorgenannten Merkmale und Vorteile der erfindungsgemäßen Linse verwiesen.

Die Erfindung wird nachfolgend anhand von in den beigeschlossenen Zeichnungen dargestellten Ausführungsbeispielen näher erläutert. In den Zeichnungen zeigen:
Fig. 1 die erfindungsgemäße Linse in einer schematischen Draufsicht;
Fig. 2 die Linse von Fig. 1 in einer schematischen Seitenansicht;
Fig. 3 Profile einer ersten und einer zweiten diffraktiven Struktur sowie das Profil einer sich aus der Überlagerung der ersten und zweiten Strukturen ergebenden Überlagerungsstruktur für die Linse von Fig. 1;
Fig. 4 die Linse von Fig. 1 mit der Überlagerungsstruktur von Fig. 3 in einem vergrößerten Halbschnitt;
Fig. 5 die Intensitätsverteilung einer mit der ersten diffraktiven Struktur von Fig. 3 ausgestatteten Linse;
Fig. 6 die Intensitätsverteilung einer mit der zweiten diffraktiven Struktur von Fig. 3 ausgestatteten Linse;
Fig. 7 die Intensitätsverteilung der erfindungsgemäßen Linse von Fig. 1;
Fig. 8 eine erste Ausführungsform des erfindungsgemäßen Verfahrens zur Herstellung der Linse von Fig. 1 in einem schematischen Blockdiagramm;
Fig. 9 eine zweite Ausführungsform des erfindungsgemäßen Verfahrens zur Herstellung der Linse von Fig. 1 in einem schematischen Blockdiagramm; und
Fig. 10 einen Vergleich der Intensitätsverteilung der erfindungsgemäßen Linse mit einer Linse nach dem Stand der Technik.

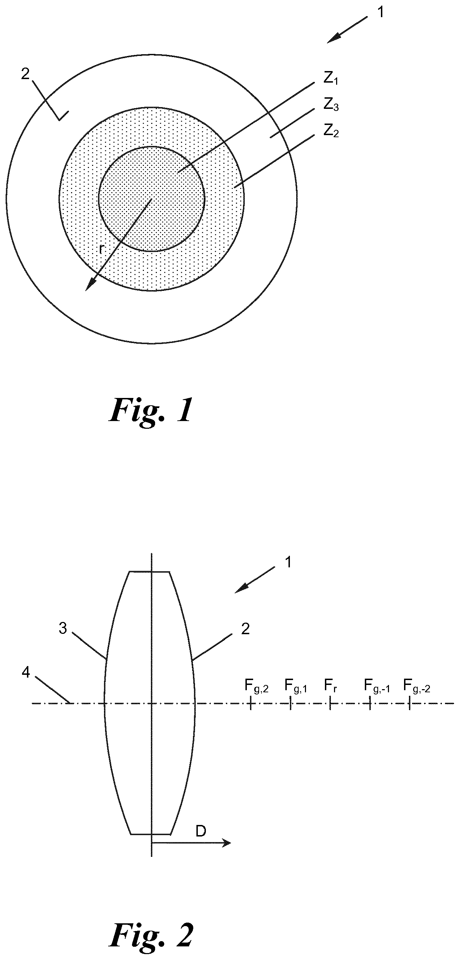

Die Fig. 1 und 2 zeigen eine Linse 1 mit einer Vorderseite 2, einer Rückseite 3 und einer optischen Achse 4. Die Linse 1 hat eine zentrale Zone Z₁ und zwei annulare Zonen Z₂, Z₃, die später noch näher erläutert werden. Die beschriebene Linse 1 wird als Intraokularlinse oder Kontaktlinse eingesetzt, kann aber auch in optischen Geräten verwendet werden.

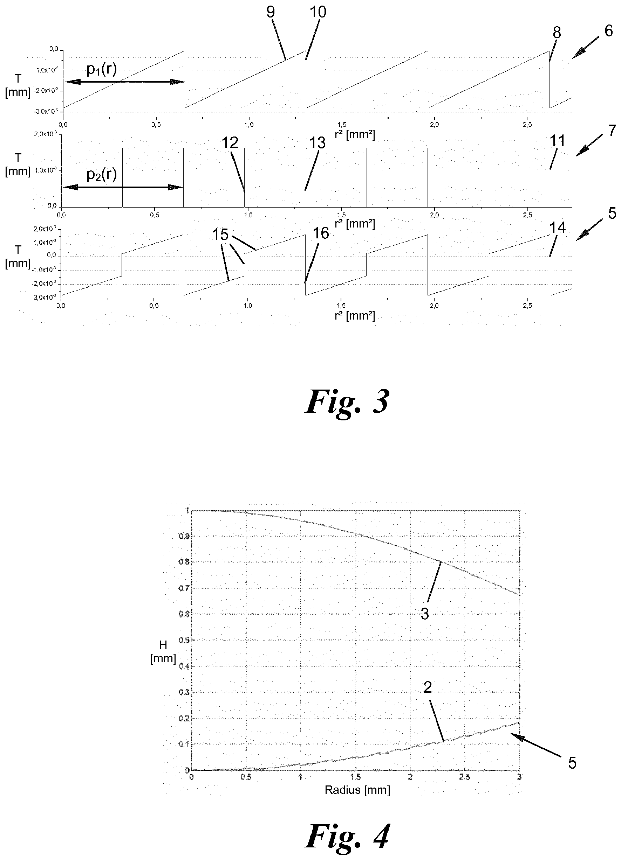

Die Linse 1 hat einen auf der optischen Achse 4 liegenden refraktiven Brennpunkt Fᵣ, der wie unten beschrieben für die Fern- oder Nahsicht eingesetzt werden kann und im Folgenden auch als Brennpunkt nullter Ordnung bezeichnet wird. In die Rück- oder Vorderseite 2, 3 der Linse 1 ist eine diffraktive Struktur 5 eingearbeitet, siehe Fig. 4, um die Linse 1 sowohl für Nah- als auch Intermediär- und Fernsicht zu adaptieren.

Die diffraktive Struktur 5 erzeugt eine Vielzahl von auf der optischen Achse 4 liegenden weiteren Brennpunkten F_{g,i}, i = ..., -2, -1, 1, 2, ..., die im Wesentlichen symmetrisch um den refraktiven Brennpunkt Fᵣ verteilt sind, wobei der refraktive Brennpunkt Fᵣ durch die Form der Linse 1 unabhängig von der aufgetragenen diffraktiven Struktur 5 gegeben ist. Die diffraktiven Brennpunkte F_{g,1}, F_{g,2} werden als Brennpunkte positiver erster bzw. zweiter Ordnung der diffraktiven Struktur 5 bezeichnet und liegen auf der optischen Achse 4 zwischen der Linse 1 und dem refraktiven Brennpunkt Fᵣ. Die diffraktiven Brennpunkte F_{g,-1}, F_{g,-2} werden als Brennpunkte negativer erster bzw. zweiter Ordnung der diffraktiven Struktur 5 bezeichnet und liegen auf der der Linse 1 abgewandten Seite des refraktiven Brennpunkts Fᵣ.

Obwohl die (Positions-)Verteilung der Brennpunkte F_{g,i} im Wesentlichen symmetrisch um den refraktiven Brennpunkt Fᵣ ist, soll die Intensitätsverteilung, die den jeweiligen Brennpunkten F_{g,i} zukommt, nicht symmetrisch sein. So sollen sich bei einer trifokalen Linse insbesondere drei größte Intensitäten ausbilden, nämlich für die Fern-, Intermediär- und Nahsicht. Dies wird erreicht, indem die diffraktive Struktur 5 wie folgt aus einer ersten und einer zweiten diffraktiven Struktur 6, 7 gebildet wird, die sich in zumindest einer der Zonen Z₁, Z₂, Z₃ überlagern.

Fig. 3 zeigt in dem oberen Diagramm das Profil 11 der ersten diffraktiven Struktur 6 (Abszisse: Radius r² [mm²]; Ordinate: Profiltiefe T [mm]). Die erste diffraktive Struktur 6 hat eine Gitterperiode p₁(r), die vom Radius r abhängig ist, genauer von r². Wird die Struktur 6 über r² als Abszissenmaßstab aufgetragen, tritt die Periode p₁ in äquidistanten Wiederholungen über der Abszisse auf.

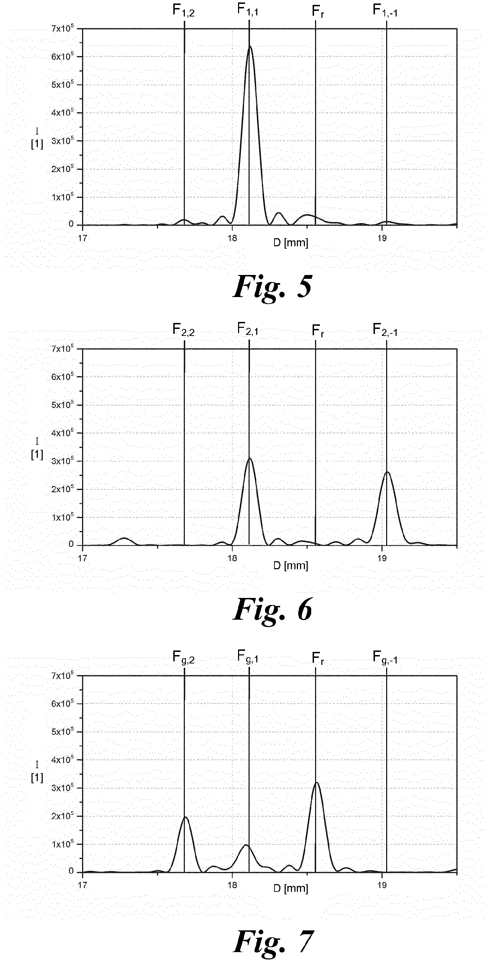

Die erste diffraktive Struktur 6 hat eine um den refraktiven Brennpunkt Fᵣ asymmetrische Intensitätsverteilung in ihren diffraktiven Brennpunkten F_{1,i}, siehe dazu später Fig. 5, und weist dazu ein Profil 8 mit, aufgetragen über dem quadrierten Radius r², asymmetrisch an- und absteigenden Flanken 9, 10 auf, wobei eine der Flanken 9, 10 senkrecht sein kann.

Fig. 3 zeigt die erste diffraktive Struktur 6 mit Sägezahnform, alternativ könnte sie jedoch auch eine Echelette- oder Kinoform aufweisen. Die Gitterperiode p₁(r) bestimmt den Abstand der von der ersten diffraktiven Struktur 6 erzeugten Brennpunkte F_{1,i} vom refraktiven Brennpunkt Fᵣ.

Fig. 3 zeigt in dem mittleren Diagramm das Profil der zweiten diffraktiven Struktur 7 (Abszisse: Radius r² [mm²]; Ordinate: Profiltiefe T [mm]). Die zweite diffraktive Struktur 7 hat eine Gitterperiode p₂(r), die vom Radius r abhängig ist, genauer von r². Wird die Struktur 7 über r² als Abszissenmaßstab aufgetragen, tritt die Periode p₂ in äquidistanten Wiederholungen über der Abszisse auf.

Die zweite diffraktive Struktur 7 hat eine um den refraktiven Brennpunkt Fᵣ im Wesentlichen symmetrische Intensitätsverteilung in ihren diffraktiven Brennpunkten F_{2,i}, wie später anhand der Fig. 5 noch ausführlicher erläutert. Die Struktur 7 weist dazu ein Profil mit zueinander, aufgetragen über dem quadratischen Radius r², im Wesentlichen symmetrisch an- und absteigenden Flanken 12, 13, die bevorzugt senkrecht sind, auf. Der Begriff "im Wesentlichen symmetrisch" umfasst dabei jeweils geringfügige Abweichungen von der Symmetrie.

Fig. 3 zeigt die zweite diffraktive Struktur 7 als binäre Struktur, d.h. mit Rechteckform. Alternativ könnte sie jedoch auch z.B. eine Trapez- oder Sinusform aufweisen. Die Gitterperiode p₂(r) bestimmt den Abstand der von der zweiten diffraktiven Struktur 7 erzeugten Brennpunkte F_{2,i} vom refraktiven Brennpunkt Fᵣ.

Fig. 5 zeigt die Intensitätsverteilung einer Linse, auf die lediglich eine erste diffraktive Struktur 6 gemäß dem obersten Diagramm von Fig. 3 aufgetragen ist (Abszisse: Distanz D von der Linse [mm]; Ordinate: Relative Intensität I [1]). Wie gezeigt ist die Intensitätsverteilung der diffraktiven Brennpunkte F_{1,i} asymmetrisch um den refraktiven Brennpunkt.

Fig. 6 zeigt die Intensitätsverteilung einer Linse 1, auf die lediglich eine zweite diffraktive Struktur 7 gemäß dem mittleren Diagramm von Fig. 3 aufgetragen ist (Abszisse: Distanz D von der Linse [mm]; Ordinate: Relative Intensität I [1]). Wie gezeigt kommt den Brennpunkten F_{2,1}, F_{2,2}, ... positiver Ordnung ein im Wesentlichen gleicher Intensitätsanteil zu wie den Brennpunkten F_{2,-1}, F_{2,-2}, ... negativer Ordnung.

Wie in Fig. 3 gezeigt sind die Gitterperioden p₁(r) und p₂(r) gleich groß, so dass sich gemäß den Fig. 5 und 6 der erste positive Brennpunkt F_{1,1} der ersten diffraktiven Struktur 6 in einem Abstand vom refraktiven Brennpunkt Fᵣ befindet, der dem Abstand des ersten positiven Brennpunkts F_{2,1} der zweiten diffraktiven Struktur 7 entspricht.

Gemäß dem untersten Diagramm von Fig. 3 wird die zusammengesetzte, "überlagerte" diffraktive Struktur 5 durch additive Überlagerung der beiden Strukturen 6, 7 erhalten (Abszisse: Radius r² [mm²]; Ordinate: Profiltiefe T [mm]). Die überlagerte diffraktive Struktur 5 hat ein Profil 14 mit an- und absteigenden Flanken 15, 16, so dass das Profil 14 der diffraktiven Struktur 5 im Wesentlichen innerhalb einer Periode monoton steigend ist. Die diffraktive Struktur 5 mit dem Profil 14 wird auf eine der Seiten 2, 3 der Linse 1 aufgebracht, siehe Fig. 4. Um die Herstellung zu erleichtern, können die Profile 8, 11, 14 abgerundete oder abgefaste Flanken 9, 10, 12, 13, 15, 16 haben.

Wie in den drei Diagrammen von Fig. 3 gezeigt, ist das Profil 8 der ersten diffraktiven Struktur 6 innerhalb der Periode p₁(r) monoton steigend und das Profil 11 der zweiten diffraktiven Struktur 7 hat in der ersten Hälfte der Periode p₂(r) ein Minimum und in der zweiten Hälfte der Periode ein Maximum. Dies führt dazu, dass das Profil 14 der überlagerten diffraktiven Struktur 5 innerhalb der Periode p₁(r), p₂(r) monoton steigend ist und somit nur ein Maximum innerhalb dieser Periode hat. In einer alternativen Ausführungsform (nicht gezeigt) könnte die zweite diffraktive Struktur 7 das Maximum auch in der ersten Hälfte der Periode p₂(r) und das Minimum in der zweiten Hälfte der Periode haben, was dann zu einer überlagerten diffraktiven Struktur 5 mit zwei Maxima pro Periode führt. Aus Fertigungsgründen ist die erstgenannte Variante mit nur einem Maximum pro Periode zu bevorzugen.

Fig. 7 zeigt die Intensitätsverteilung der Linse 1, in die die diffraktive Struktur 5 aus den addierten bzw. überlagerten Strukturen 6, 7 eingearbeitet ist (Abszisse: Distanz D von der Linse [mm]; Ordinate: Relative Intensität I [1]). Der refraktive Brennpunkt Fᵣ wird hier für die Fernsicht eingesetzt, der erste positive diffraktive Brennpunkt F_{g,1} entspricht in seiner Position den Brennpunkten F_{1,1}, F_{2,1} erster (positiver) Ordnung der diffraktiven Strukturen 6, 7 und wird für die Intermediärsicht verwendet, und der zweite positive diffraktive Brennpunkt F_{g,2} entspricht in seiner Position den Brennpunkten F_{1,2}, F_{2,2} zweiter (positiver) Ordnung der diffraktiven Strukturen 6, 7 und wird für die Nahsicht verwendet. Alternativ könnten die Steigungen der Flanken 9, 10, 12, 13 jeweils umgekehrt werden, wodurch dann z.B. der refraktive Brennpunkt Fᵣ für die Nahsicht und die diffraktiven Brennpunkte erster Ordnung F_{1,1}, F_{2,1} bzw. zweiter Ordnung F_{1,2}, F_{2,2} für die Intermediär- bzw. Fernsicht verwendet werden könnten.

Die beiden Strukturen 6, 7 können jeweils entweder auf die ganze Oberfläche der Seite 2, 3 der Linse 1 aufgebracht werden oder lediglich zonal, wie in Fig. 1 dargestellt. So kann z.B. die kombinierte Struktur 5 lediglich in der innersten Zone Z₁, d.h. im zentralen Bereich, der Linse 1 aufgebracht werden. In einer umgebenden annularen Zone Z₂ kann nur eine der beiden Strukturen 6, 7 aufgetragen sein, und in einer äußersten annularen Zone Z₃ keine der Strukturen 5, 6, 7. Dadurch wird eine von der Pupillengröße abhängige Intensitätsverteilung erreicht, wobei die Gewichtung der Intensität am refraktiven Brennpunkt Fᵣ mit zunehmender Pupillengröße zunimmt.

Alternativ oder zusätzlich kann dieser Effekt durch eine Apodisierung der Strukturen 5, 6, 7 erreicht werden. Dies bedeutet, dass die Tiefe T der Profile 8, 11, 14 der Strukturen 5, 6, 7 mit zunehmendem Linsenradius r abnimmt (nicht in Fig. 3 gezeigt).

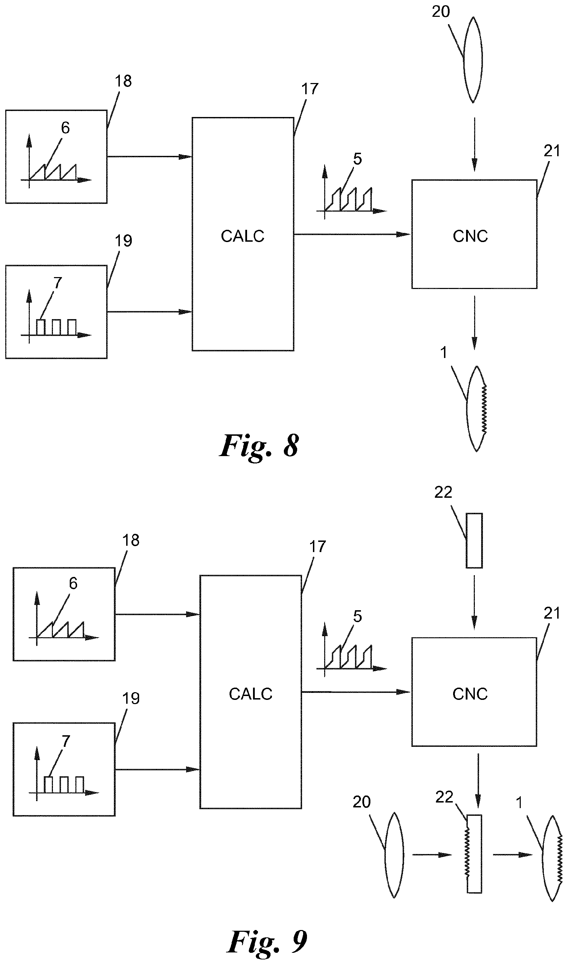

Die Fig. 8 und 9 zeigen zwei Varianten des Verfahrens zur Herstellung der multifokalen Linse 1. Zur Berechnung der diffraktiven Struktur 5, auch "Überlagerungsstruktur" 5 genannt, werden in einer Recheneinheit 17 die erste diffraktive Struktur 6, die eine um den refraktiven Brennpunkt Fᵣ asymmetrische Intensitätsverteilung hat, und die zweite diffraktive Struktur 7, die eine um den refraktiven Brennpunkt Fᵣ im Wesentlichen symmetrische Intensitätsverteilung hat, überlagert, z.B. addiert. Die Strukturen 6, 7 können der Recheneinheit 17 mittels Speichern 18, 19 zur Verfügung gestellt werden oder in der Recheneinheit 17 selbst berechnet oder ermittelt werden.

Fig. 8 zeigt eine erste Variante des Herstellungsverfahrens, bei der die in der Recheneinheit 17 berechnete Überlagerungsstruktur 5 in einen Linsenrohling 20 eingearbeitet wird, z.B. durch eine spanabhebende Bearbeitung wie Drehen in einer Drehmaschine 21, um so die multifokale Linse 1 herzustellen. Dabei wird beispielsweise der Linsenrohling 20 um seine optische Achse gedreht und das Drehwerkzeug der Drehmaschine 21 arbeitet während der Drehung des Linsenrohlings 20 die Überlagerungsstruktur 5 in den Linsenrohling 20 ein. Nach der spanabhebenden Bearbeitung kann die Linse 1 optional poliert werden.

Der Linsenrohling 20 könnte aber auch lediglich ein verarbeitungsfähiges Ausgangsmaterial für einen 3D-Drucker sein, und das Einarbeiten der Überlagerungsstruktur 5 in den Linsenrohling 5 erfolgt dann durch 3D-Drucken des Ausgangsmaterials 20 zu der multifokalen Linse 1.

Fig. 9 zeigt eine zweite Variante des Herstellungsverfahrens, bei der die in der Recheneinheit berechnete Überlagerungsstruktur 5 zunächst als Negativ in einen Formrohling 22 eingearbeitet wird, z.B. wieder mittels einer Drehmaschine 21 oder eines 3D-Druckers. Anschließend wird ein Linsenmaterial 20 mit dem Formrohling 22 in Kontakt gebracht, um so die multifokale Linse 1 herzustellen. Das Linsenmaterial 20 kann z.B. bereits zu einem Linsenrohling vorgefertigt sein, in welchen die Überlagerungsstruktur 5 mittels des Formrohlings 22 als "Stempel" eingepresst oder eingeprägt wird. Alternativ kann das Linsenmaterial 20 in flüssigem bzw. viskosem Zustand vorliegen und auf den Formrohling gegossen werden, z.B. in einer Form. Daraufhin wird das Linsenmaterial 20 ausgehärtet, z.B. mittels Licht- oder Wärmezufuhr.

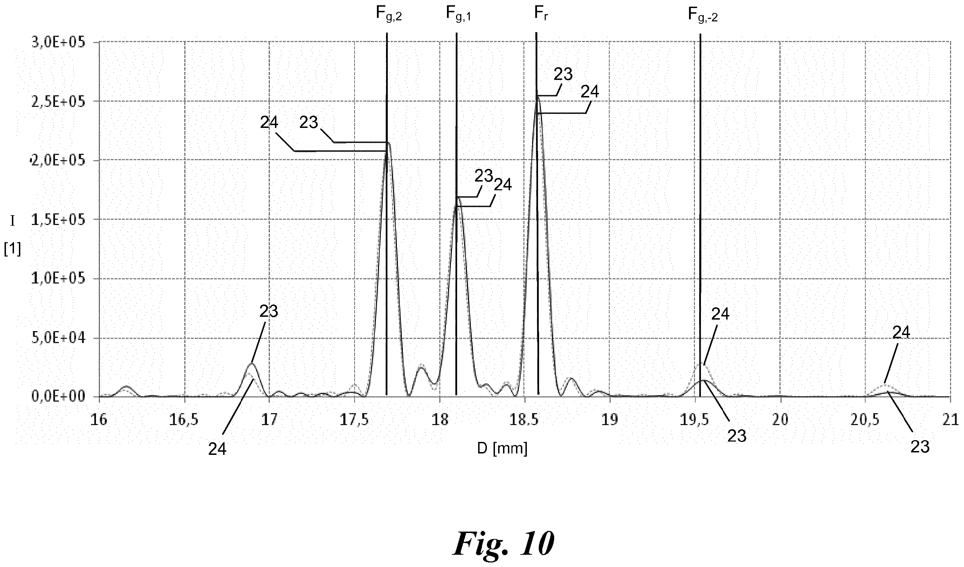

Fig. 10 zeigt einen Vergleich des Intensitätsverlaufs 23 der hier vorgestellten Linse 1 mit dem Intensitätsverlauf 24 einer Linse gemäß dem Stand der Technik (Abszisse: Distanz D von der Linse [mm] ; Ordinate: Relative Intensität I [1]). Die Vergleichslinse gemäß dem Stand der Technik ist mit einer Struktur ausgestattet, bei der zwei diffraktive Strukturen mit asymmetrischen Intensitätsverteilungen (z.B. Strukturen mit Sägezahnform) überlagert werden, wobei der Brennpunkt erster Ordnung der ersten diffraktiven Struktur mit dem Brennpunkt zweiter Ordnung der zweiten diffraktiven Struktur zusammenfällt.

Dadurch ergibt sich ein ähnlicher Verlauf der Intensitätsverteilungen im Bereich des refraktiven Brennpunkts Fᵣ. Aus Fig. 10 ist jedoch gut erkennbar, dass die Linse 1 gemäß dem Stand der Technik im Bereich des zweiten negativen Brennpunkts F_{g,-2} der kombinierten diffraktiven Struktur 5 (bzw. des ersten negativen Brennpunkts F_{2,-1} der zweiten diffraktiven Struktur 7) größere Intensitätswerte aufweist. Im Gegensatz dazu sind bei der hier vorgestellten Linse 1 nicht nutzbare Intensitäten aus negativen Ordnungen in nutzbare positive Ordnungen verschoben, wie anhand der deutlich erhöhten Intensitäten des Verlaufs 17 an den Brennpunkten F_{g,1} und F_{g,2} sowie der deutlich reduzierten Intensität des Verlaufs 23 am Brennpunkt F_{g,-2} erkennbar ist. Für den Nutzer der beschriebenen Linse 1 ergibt sich somit ein farbintensiveres und kontrastreicheres Bild als mit Linsen gemäß dem Stand der Technik.

Die Erfindung ist demgemäß nicht auf die dargestellten Ausführungsformen beschränkt, sondern umfasst alle Varianten, Modifikationen und Kombinationen derselben, die in den Rahmen der angeschlossenen Ansprüche fallen.

## Patentansprüche

1. Multifokale Intraokular- oder Kontaktlinse, mit einem refraktiven Brennpunkt (Fᵣ) und einer ersten und einer zweiten diffraktiven Struktur (6, 7), die sich zumindest teilweise überlagern, wobei die beiden diffraktiven Strukturen (6, 7) unterschiedlich sind, **dadurch gekennzeichnet, dass** ein Brennpunkt erster Ordnung (F_{1,1}) der ersten diffraktiven Struktur (6) mit einem Brennpunkt erster Ordnung (F_{2,1}) der zweiten diffraktiven Struktur (7) zusammenfällt.

2. Multifokale Intraokular- oder Kontaktlinse nach Anspruch 1, **dadurch gekennzeichnet, dass** die beiden diffraktiven Strukturen (6, 7) unterschiedliche Intensitätsverteilungen haben.

3. Multifokale Intraokular- oder Kontaktlinse nach Anspruch 1 oder 2, **dadurch gekennzeichnet, dass** die erste diffraktive Struktur (6) eine um den refraktiven Brennpunkt (Fᵣ) asymmetrische Intensitätsverteilung und die zweite diffraktive Struktur (7) eine um den refraktiven Brennpunkt (Fᵣ) im Wesentlichen symmetrische Intensitätsverteilung hat.

4. Multifokale Intraokular- oder Kontaktlinse nach einem der Ansprüche 1 bis 3, **dadurch gekennzeichnet, dass** die Profile (8, 11) der beiden diffraktiven Strukturen (6, 7) eine gleiche Periodenlänge (p₁, p₂) haben, wobei das Profil (8) der ersten diffraktiven Struktur (6) innerhalb der Periode (p₁) monoton steigend ist und das Profil (11) der zweiten diffraktiven Struktur (7) in der ersten Hälfte der Periode (p₂) ein Minimum und in der zweiten Hälfte der Periode ein Maximum hat.

5. Multifokale Intraokular- oder Kontaktlinse nach einem der Ansprüche 1 bis 4, **dadurch gekennzeichnet, dass** die erste diffraktive Struktur (6) ein Profil (8) mit Sägezahn-, Echelette- oder Kinoform hat.

6. Multifokale Intraokular- oder Kontaktlinse nach einem der Ansprüche 1 bis 5, **dadurch gekennzeichnet, dass** die zweite diffraktive Struktur (7) ein Profil (11) mit Rechteck-, Trapez- oder Sinusform hat.

7. Multifokale Intraokular- oder Kontaktlinse nach einem der Ansprüche 1 bis 6, **dadurch gekennzeichnet, dass** zumindest eines der Profile (8, 11, 14) abgerundete oder abgefaste Flanken (9, 10, 12, 13) hat.

8. Multifokale Linse nach einem der Ansprüche 1 bis 7, **dadurch gekennzeichnet, dass** die beiden diffraktiven Strukturen (6, 7) in radialer Richtung der Linse (1) apodisiert sind, bevorzugt unterschiedlich stark.

9. Verfahren zum Herstellen einer multifokalen Intraokular- oder Kontaktlinse nach Anspruch 1, **gekennzeichnet durch** die Schritte:
a) Bereitstellen eines Linsenrohlings (20) mit einem refraktiven Brennpunkt (Fᵣ);
b) Berechnen einer Überlagerungsstruktur (5) aus einer ersten diffraktiven Struktur (6) und einer zweiten diffraktiven Struktur (7), wobei die beiden diffraktiven Strukturen (6, 7) unterschiedlich sind und wobei ein Brennpunkt erster Ordnung (F_{1,1}) der ersten diffraktiven Struktur (6) mit einem Brennpunkt erster Ordnung (F_{2,1}) der zweiten diffraktiven Struktur (7) zusammenfällt; und
c) Einarbeiten der Überlagerungsstruktur (5) in den Linsenrohling (20) zum Herstellen der multifokalen Linse (1).

10. Verfahren nach Anspruch 9, **dadurch gekennzeichnet, dass** das Einarbeiten der Überlagerungsstruktur (5) in den Linsenrohling (20) mittels Drehens durchgeführt wird.

11. Verfahren zum Herstellen einer multifokalen Intraokular- oder Kontaktlinse nach Anspruch 1, **gekennzeichnet durch** die Schritte:
a) Bereitstellen eines Formrohlings (22);
b) Berechnen einer Überlagerungsstruktur (5) aus einer ersten diffraktiven Struktur (6) und einer zweiten diffraktiven Struktur (7), wobei die beiden diffraktiven Strukturen (6, 7) unterschiedlich sind und wobei ein Brennpunkt erster Ordnung (F_{1,1}) der ersten diffraktiven Struktur (6) mit einem Brennpunkt erster Ordnung (F_{2,1}) der zweiten diffraktiven Struktur (7) zusammenfällt;
c) Einarbeiten der Überlagerungsstruktur (5) als Negativ in den Formrohling (22); und
d) Herstellen der multifokalen Linse (1) durch Inkontaktbringen eines Linsenmaterials (20) mit dem Formrohling (22), wobei dem Linsenmaterial (20) auch ein refraktiver Brennpunkt (Fᵣ) verliehen wird.

12. Verfahren nach Anspruch 11, **dadurch gekennzeichnet, dass** das Inkontaktbringen durch Gießen des Linsenmaterials (20) auf den Formrohling (22) und Aushärten des Linsenmaterials (20) erfolgt.

13. Verfahren nach einem der Ansprüche 9 bis 12, **dadurch gekennzeichnet, dass** die erste diffraktive Struktur (6) eine um den refraktiven Brennpunkt (Fᵣ) asymmetrische Intensitätsverteilung und die zweite diffraktive Struktur (7) eine um den refraktiven Brennpunkt (Fᵣ) im Wesentlichen symmetrische Intensitätsverteilung hat.

14. Verfahren nach einem der Ansprüche 9 bis 13, **dadurch gekennzeichnet, dass** die Profile (8, 11) der beiden diffraktiven Strukturen (6, 7) eine gleiche Periodenlänge (p₁, p₂) haben, wobei das Profil (8) der ersten diffraktiven Struktur (6) innerhalb der Periode (p₁) monoton steigend ist und das Profil (11) der zweiten diffraktiven Struktur (7) in der ersten Hälfte der Periode (p₂) ein Minimum und in der zweiten Hälfte der Periode (p₂) ein Maximum hat.

15. Verfahren nach einem der Ansprüche 9 bis 14, **dadurch gekennzeichnet, dass** zumindest eine der beiden diffraktiven Strukturen (6, 7) abgerundete oder abgefaste Flanken hat.

16. Verfahren nach einem der Ansprüche 9 bis 15, **dadurch gekennzeichnet, dass** die beiden diffraktiven Strukturen (6, 7) in radialer Richtung der Linse (1) apodisiert sind, bevorzugt unterschiedlich stark.

## Claims

1. A multifocal intraocular or contact lens, having a refractive focal point (Fᵣ) and a first and a second diffractive structure (6, 7), which at least partially overlap,
wherein the two diffractive structures (6, 7) are different,
**characterised in that**
a first order focal point (F_{1,1}) of the first diffractive structure (6) coincides with a first order focal point (F_{2,1}) of the second diffractive structure (7).

2. The multifocal intraocular or contact lens according to claim 1,
**characterised in that** the two diffractive structures (6, 7) have different intensity distributions.

3. The multifocal intraocular or contact lens according to claim 1 or 2,
**characterised in that** the first diffractive structure (6) has an intensity distribution that is asymmetric around the refractive focal point (Fᵣ) and the second diffractive structure (7) has an intensity distribution that is substantially symmetric around the refractive focal point (Fᵣ).

4. The multifocal intraocular or contact lens according to one of claims 1 to 3, **characterised in that** the profiles (8, 11) of the two diffractive structures (6, 7) have the same period length (p₁, p₂), wherein the profile (8) of the first diffractive structure (6) is monotonically rising within the period (p₁) and the profile (11) of the second diffractive structure (7) has a minimum in the first half of the period (p₂) and a maximum in the second half of the period.

5. The multifocal intraocular or contact lens according to one of claims 1 to 4, **characterised in that** the first diffractive structure (6) has a profile (8) with saw-tooth, echelette or kinoform shape.

6. The multifocal intraocular or contact lens according to one of claims 1 to 5, **characterised in that** the second diffractive structure (7) has a profile (11) with rectangular, trapezoidal or sinusoidal shape.

7. The multifocal intraocular or contact lens according to one of claims 1 to 6, **characterised in that** at least one of the profiles (8, 11, 14) has rounded-off or bevelled flanks (9, 10, 12, 13).

8. The multifocal lens according to one of claims 1 to 7,
**characterised in that** the two diffractive structures (6, 7) are apodized, preferably at different strengths, in the radial direction of the lens (1).

9. A method for producing a multifocal intraocular or contact lens according to claim 1, **characterised by** the steps:
a) providing a lens blank (20) with a refractive focal point (Fᵣ);
b) calculating an overlapping structure (5) from a first diffractive structure (6) and a second diffractive structure (7), wherein the two diffractive structures (6, 7) are different, and wherein a first order focal point (F_{1,1}) of the first diffractive structure (6) coincides with a first order focal point (F_{2,1}) of the second diffractive structure (7);
c) incorporating the overlapping structure (5) into the lens blank (20) to produce the multifocal lens (1).

10. The method according to claim 9, **characterised in that** the incorporation of the overlapping structure (5) into the lens blank (20) is realised by means of rotation.

11. A method for producing a multifocal intraocular or contact lens according to claim 1, **characterised by** the steps:
a) providing a moulded blank (22);
b) calculating an overlapping structure (5) from a first diffractive structure (6) and a second diffractive structure (7), wherein the two diffractive structures (6, 7) are different, and wherein a first order focal point (F_{1,1}) of the first diffractive structure (6) coincides with a first order focal point (F_{2,1}) of the second diffractive structure (7);
c) incorporating the overlapping structure (5) as a negative into the moulded blank (22); and
d) producing the multifocal lens (1) by bringing a lens material (20) into contact with the moulded blank (22), wherein a refractive focal point (Fᵣ) is also given to the lens material (20).

12. The method according to claim 11, **characterised in that** the bringing into contact is realised by casting the lens material (20) onto the moulded blank (22) and hardening of the lens material (20).

13. The method according to one of claims 9 to 12, **characterised in that** the first diffractive structure (6) has an intensity distribution that is asymmetric around the refractive focal point (Fᵣ) and the second diffractive structure (7) has an intensity distribution that is substantially symmetric around the refractive focal point (Fᵣ).

14. The method according to one of claims 9 to 13, **characterised in that** the profiles (8, 11) of the two diffractive structures (6, 7) have the same period length (p1, p2), wherein the profile (8) of the first diffractive structure (6) is monotonically rising within the period (p1) and the profile (11) of the second diffractive structure (7) has a minimum in the first half of the period (p₂) and a maximum in the second half of the period (p₂).

15. The method according to one of claims 9 to 14, **characterised in that** at least one of the two diffractive structures (6, 7) has rounded-off or bevelled flanks.

16. The method according to one of claims 9 to 15, **characterised in that** the two diffractive structures (6, 7) are apodized, preferably at different strengths, in the radial direction of the lens (1).

## Revendications

1. Lentille multifocale intraoculaire ou de contact avec un point focal réfractif (Fᵣ) et une première et une deuxième structure diffractive (6,7) qui se chevauchent au moins partiellement, les deux structures diffractives (6,7) étant différentes, ladite lentille étant **caractérisée en ce que** un point focal d'un premier ordre (F_{1,1}) de la première structure diffractive (6) coïncide avec un point focal de premier ordre (F_{2,1}) de la deuxième structure diffractive (7).

2. Lentille multifocale intraoculaire ou de contact selon la revendication 1 **caractérisée en ce que** les deux structures diffractives (6,7) ont des distributions d'intensité différentes.

3. Lentille multifocale intraoculaire ou de contact selon la revendication 1 ou 2 **caractérisée en ce que** la première structure diffractive (6) a une distribution d'intensité asymétrique autour du point focal réfractif (Fᵣ) et la deuxième structure diffractive (7) a une distribution d'intensité sensiblement symétrique autour du point focal réfractif (Fᵣ).

4. Lentille multifocale intraoculaire ou de contact selon une des revendications 1 à 3 **caractérisée en ce que** les profils (8,11) des deux structures diffractives (6,7) ont une même longueur de période (p₁,p₂), le profil (8) de la première structure diffractive (6) dans la période (p₁) étant à croissance monotone et le profil (11) de la deuxième structure diffractive (7) ayant un minimum dans la première moitié de la période (p₂) et un maximum dans la deuxième moitié de la période.

5. Lentille multifocale intraoculaire ou de contact selon une des revendications 1 à 4 **caractérisée en ce que** la première structure diffractive (6) a un profil (8) en dents de scie, échelet ou kinoforme.

6. Lentille multifocale intraoculaire ou de contact selon une des revendications 1 à 5 **caractérisée en ce que** la deuxième structure diffractive (7) a un profil (11) de forme rectangulaire, trapézoïdale ou sinusoïdale.

7. Lentille multifocale intraoculaire ou de contact selon une des revendications 1 à 6 **caractérisée en ce qu'**au moins un des profils (8, 11, 14) a des flancs arrondis ou en biseau (9, 10, 12, 13).

8. Lentille multifocale intraoculaire ou de contact selon une des revendications 1 à 7 **caractérisée en ce que** les deux structures diffractives (6,7) sont apodisées dans le sens radial de la lentille (1), de préférence selon une intensité différente.

9. Procédé pour la fabrication d'une lentille multifocale intraoculaire ou de contact selon la revendication 1 **caractérisé par** les étapes suivantes :
a) Mise à disposition d'une ébauche de lentille (20) avec un point focal réfractif (Fᵣ) ;
b) Calcul d'une structure superposée (5) constituée d'une première structure diffractive (6) et d'une deuxième structure diffractive (7), les deux structures diffractives (6,7) étant différentes et un point focal de premier ordre (F_{1,1}) de la première structure diffractive (6) coïncidant avec un point focal de premier ordre (F_{2,1}) de la deuxième structure diffractive (7) ; et
c) Intégration de la structure superposée (5) comme négatif dans l'ébauche de lentille (22) pour fabriquer une lentille multifocale (1).

10. Procédé selon la revendication 9 **caractérisé en ce que** l'intégration de la structure superposée (5) dans l'ébauche de lentille (22) est exécutée par tournage.

11. Procédé pour la fabrication d'une lentille multifocale intraoculaire ou de contact selon la revendication 1, **caractérisé par** les étapes suivantes :
a) Mise à disposition d'une ébauche de lentille (22)
b) Calcul d'une structure superposée (5) constituée d'une première structure diffractive (6) et d'une deuxième structure diffractive (7), les deux structures diffractives (6,7) étant différentes et un point focal de premier ordre (F_{1,1}) de la première structure diffractive (6) coïncidant avec un point focal de premier ordre (F_{2,1}) de la deuxième structure diffractive (7) ;
c) Intégration de la structure superposée (5) comme négatif dans l'ébauche de lentille (22) et
d) Fabrication d'une lentille multifocale (1) en mettant en contact un matériau de lentille (20) avec une ébauche de lentille (22), un point focal réfractif (Fᵣ) étant aussi conféré au matériau de lentille (20)

12. Procédé selon la revendication 11, **caractérisé en ce que** la mise en contact se fait en versant le matériau de lentille (20) sur l'ébauche de lentille (22) et par durcissement du matériau de lentille (20).

13. Procédé selon une des revendications 9 à 12, **caractérisé en ce que** la première structure diffractive (6) a une distribution d'intensité asymétrique autour du point focal réfractif (Fᵣ) et la deuxième structure diffractive (7) a une distribution d'intensité sensiblement symétrique autour du point focal réfractif (Fᵣ).

14. Procédé selon une des revendications 9 à 13, **caractérisé en ce que** les profils (8,11) des deux structures diffractives (6,7) ont une même longueur de période (p₁,p₂), le profil (8) de la première structure diffractive (6) dans la période (p₁) étant à croissance monotone et le profil (11) de la deuxième structure diffractive (7) ayant un minimum dans la première moitié de la période (p₂) et un maximum dans la deuxième moitié de la période (p₂).

15. Procédé selon une des revendications 9 à 14, **caractérisé en ce qu'**au moins une des deux structures diffractives (6,7) a des flancs arrondis ou en biseau.

16. Procédé selon une des revendications 9 à 15, **caractérisé en ce que** les deux structures diffractives (6,7) sont apodisées dans le sens radial de la lentille (1), de préférence, selon une intensité différente.
